(19) Europäisches Patentamt
European Patent Office
Office européen des brevets

(11) **EP 4 017 554 B1**

(12) **EUROPÄISCHE PATENTSCHRIFT**

(45) Veröffentlichungstag und Bekanntmachung des Hinweises auf die Patenterteilung:
**04.12.2024 Patentblatt 2024/49**

(21) Anmeldenummer: **20761217.7**

(22) Anmeldetag: **21.08.2020**

(51) Internationale Patentklassifikation (IPC):
**A61M 1/16** (2006.01) **A61M 1/36** (2006.01)

(52) Gemeinsame Patentklassifikation (CPC):
**A61M 1/1607; A61M 1/1609; A61M 1/1656; A61M 1/3609;** A61M 2205/3306; A61M 2205/3317; A61M 2205/3372

(86) Internationale Anmeldenummer:
**PCT/EP2020/073532**

(87) Internationale Veröffentlichungsnummer:
**WO 2021/037744 (04.03.2021 Gazette 2021/09)**

(54) **VORRICHTUNG ZUR EXTRAKORPORALEN BLUTBEHANDLUNG UND VERFAHREN ZUR BILANZREGELUNG EINER DIALYSIERFLÜSSIGKEIT BEI EINER EXTRAKORPORALEN BLUTBEHANDLUNG**

DEVICE FOR EXTRACORPOREAL BLOOD TREATMENT AND METHOD FOR THE BALANCE CONTROL OF A DIALYSIS LIQUID IN AN EXTRACORPOREAL BLOOD TREATMENT

DISPOSITIF POUR TRAITEMENT EXTRACORPOREL DU SANG ET PROCÉDÉ DE RÉGULATION DU BILAN D'UN LIQUIDE DE DIALYSE LORS D'UN TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.08.2019 DE 102019122704**

(43) Veröffentlichungstag der Anmeldung:
**29.06.2022 Patentblatt 2022/26**

(73) Patentinhaber: **B. Braun Avitum AG**
**34212 Melsungen (DE)**

(72) Erfinder: **JANIK, Waldemar**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
**WO-A1-2018/114277      DE-A1- 102017 116 097**
**US-A- 5 342 527**

**Beschreibung**

Technisches Gebiet

Die vorliegende Offenbarung betrifft eine Vorrichtung zur extrakorporalen Blutbehandlung, insbesondere zur Hämodialyse, und ein Verfahren zur Bilanzregelung einer Dialysierflüssigkeit bei einer extrakorporalen Blutbehandlung.

Hintergrund der Erfindung

Bei der extrakorporalen Blutbehandlung (z.B. Hämodialyse) wird das Blut eines Patienten durch einen Dialysator geleitet, durch den zur gleichen Zeit eine Dialysierflüssigkeit geleitet wird. Im Dialysator werden Blut und Dialysierflüssigkeit über eine semipermeable Membran in Kontakt gebracht, so dass ein Stoffaustausch zwischen dem Patientenblut und der Dialysierflüssigkeit stattfinden kann. Ziel einer derartigen Dialysebehandlung ist es, bei einem niereninsuffizienten Patienten das Blut zu entgiften und auch überschüssiges Wasser aus dem Körper zu entfernen.

Die Dialysierflüssigkeit besteht aus hochreinem Wasser, einer basischen ersten Komponente (Natriumhydrogencarbonat (NaHCOs)) sowie einer sauren zweiten Komponente. Letztere setzt sich üblicherweise aus Natriumchlorid (NaCl), Kaliumchlorid (KCl), Magnesiumchlorid ($MgCl_2$), Calciumchlorid ($CaCl_2$), Essigsäure ($CH_3COOH$) und Glucose zusammen. In der Regel werden zur Herstellung bzw. Proportionierung der Dialysierflüssigkeit Dosierpumpen und Leitfähigkeitssonden eingesetzt. Eine Sonde misst dabei die Leitfähigkeit nach Zugabe des Natriumhydrogencarbonats mittels einer ersten Dosierpumpe. Eine weitere Sonde erfasst die Leitfähigkeit der gesamten Dialysierflüssigkeit, nachdem auch die saure Komponente mittels einer weiteren Dosierpumpe zugegeben worden ist. Bei der leitfähigkeitsgesteuerten Proportionierung werden die Zugabemengen anhand der gemessenen Leitfähigkeiten reguliert.

Bei der volumetrischen Proportionierung dienen die Leitfähigkeitssonden lediglich zur Kontrolle. Die Proportionierung erfolgt hier direkt über die Dosierpumpenförderraten, weswegen hierbei die genaue Zusammensetzung der eingesetzten Komponenten bekannt sein muss.

Die richtige Zusammensetzung der Dialysierflüssigkeit ist für das Wohlbefinden und die Lebenserwartung eines Patienten von großer Bedeutung. Insbesondere Natrium spielt hierbei eine bedeutende Rolle, da es sowohl in der Dialysierflüssigkeit als auch im Blutplasma das am häufigsten vorkommende Kation ist. Bei niereninsuffizienten Patienten ist die Regulierung des Wasser- und Elektrolythaushalts über die Nieren stark eingeschränkt. Aus diesem Grund sind die Patienten darauf angewiesen, ihre Wasser- und Natriumaufnahme auf ein Mindestmaß zu beschränken. Natrium wird insbesondere in Form von Kochsalz (NaCl) aufgenommen. Eine erhöhte Natriumaufnahme gleicht ein Dialysepatient aus, indem er eine entsprechende Menge Flüssigkeit aufnimmt.

Eine erhöhte Flüssigkeitsaufnahme führt allerdings dazu, dass dem Patienten während der nächsten Dialysebehandlung mehr überschüssige Flüssigkeit durch Ultrafiltration entzogen werden muss. Studien haben jedoch gezeigt, dass eine erhöhte Ultrafiltrationsrate mit einer höheren Sterblichkeitsrate einhergeht (Flythe, J. E.; Kimmel, S. E., Brunelli, S. M.: "Rapid fluid removal during dialysis is associated with cardiovascular morbidity and mortality", Kidney International, 2010, 79, 250-257).

Während der Dialyse können hohe Ultrafiltrationsraten zudem symptomatische Blutdruckabfälle verursachen (Saran, R.; Bragg-Gresham, J.; Levin, N.; Twardowski, Z.; Wizemann, V.; Saito, A.; Kimata, N., Gillespie, B.: "Longer treatment time and slower ultrafiltration in hemodialysis: Associations with reduced mortality in the DOPPS", Kidney International, 2006, 69, 1222-1228).

Um letztere zu behandeln oder zu vermeiden, werden dem Patienten NaCl-Injektionen verabreicht oder wird der Patient gegen eine bzgl. Natrium recht hoch konzentrierte Dialysierflüssigkeit dialysiert. Allerdings führt beides wieder zu einer erhöhten Wasseraufnahme. Ein Teufelskreis beginnt.

Allerdings ist eine zu geringe Konzentration an Natrium in der Dialysierflüssigkeit für den Patienten ebenfalls nachteilig. Im Patienten stehen der intrazelluläre und der extrazelluläre Raum in einem osmotischen Gleichgewicht zueinander. Eine geringe Natriumkonzentration in der Dialysierflüssigkeit würde aufgrund von Diffusionsprozessen innerhalb des Dialysators zu einem Absinken der extrazellulären Osmolarität (osmotischen Konzentration) führen. Da aber beide Räume im Gleichgewicht stehen, würde Wasser aus dem intrazellulären Raum in den extrazellulären Raum diffundieren. Infolgedessen würden die Zellen schrumpfen, was zu Schwindel, Kopfschmerzen, Übelkeit, Krämpfen und ebenfalls zu Blutdruckabfällen führen kann.

Aus oben genannten Gründen ist es deshalb erstrebenswert, die Natrium-konzentration in der Dialysierflüssigkeit individuell so anzupassen, dass die Plasma-Natrium-Konzentration des Patienten während der Behandlung möglichst wenig und bestenfalls gar nicht geändert wird. Eine derartige Dialyse wird als isonaträmisch bezeichnet. (de Paula, F. M.; Peixoto, A. J.; Pinto, L. V.; Dorigo, D.; Patricio, P. J. M., Santos, S. F. F.: "Clinical consequences of an individualized dialysate sodium Prescription in hemodialysis patients", Kidney International, 2004, 66, 1232-1238; und Basile, C., Lomonte, C.: "It is Time to Individualize the Dialysate Sodium Prescription", Seminars in Dialysis, 2016, 29, 24-27).

Am einfachsten wäre es, dem Patienten vor jeder Behandlung eine Blutprobe zu entnehmen und diese zu analysieren, um die Natriumkonzentration in der Dialysierflüssigkeit auf dieser Basis einzustellen. Allerdings ist diese Vorgehensweise sehr zeitintensiv und kostspielig sowie mit einem hohen apparativen Aufwand verbunden. Aus diesem Grund wird in vielen Dialysezentren mit einer standardisierten Zusammensetzung der Dialysierflüssigkeit dialysiert, obwohl die Plasma-Natrium-Konzentration von Patient zu Patient unterschiedlich ausfallen kann (Mendoza, J. M.; Sun, S.; Chertow, G. M.; Moran, J.; Dass, S., Schiller, B.: "Dialysate Sodium and Sodium gradient in maintenance hemodialysis: a neglected sodium restriction approach?", Nephrol Dial Transplant, 2011, 26, 1281-1287).

Eine Dialysevorrichtung mit geregelter Zusammensetzung der Dialysierflüssigkeit wird in der EP 0 097 366 A2 offenbart. Bei dieser Dialysevorrichtung wird der Elektrolytgehalt der Dialysierflüssigkeit bevor diese in den Dialysator eintritt und nachdem sie den Dialysator wieder verlässt mittels zweier Detektoren erfasst und wird basierend auf dem Unterschied der erhaltenen Messwerte die Zusammensetzung der Dialysierflüssigkeit geregelt.

Eine Dialysevorrichtung zur Durchführung einer isonaträmischen Dialyse und ein Verfahren zur Natriumbilanzierung im Dialysatkreislauf ist aus der WO 2010/ 112 223 A1 bekannt. Im Speziellen wird in einem Dialysatkreislauf die Leitfähigkeit der Dialysierflüssigkeit vor und nach dem Dialysator bestimmt und miteinander verglichen. Als weitere Parameter werden zudem die Konzentrationen von Bicarbonat und Kalium im Blut des Patienten benötigt, die zuvor mithilfe eines externen Messgerätes bestimmt werden müssen.

Die DE 699 16 053 T2 beschreibt ein Verfahren zur Bestimmung von Abfallprodukten in einer aus einem Dialysator austretenden Dialysierflüssigkeit, bei dem mittels einer geeigneten Messzelle spektralphotometrisch der Gehalt einer oder einer Kombination von Substanzen in der Dialysierflüssigkeit bestimmt wird.

Kuhlmann, U.; Maierhofer, A.; Canaud, B.; Hoyer, J., Gross, M., "Zero Diffusive Sodium Balance in Hemodialysis Provided by an Algorithm-Based Electrolyte Balancing Controller: A Proof of Principle Clinical Study", Artificial Organs, Wiley, 2018, beschreiben einen Algorithmus zur Steuerung der Natriumkonzentration in der Dialysierflüssigkeit einer Hämodialyse oder Hämodiafiltration basierend auf Leitfähigkeitsmessungen der Dialysierflüssigkeit vor und nach dem Dialysator.

Eine Vorrichtung und ein Verfahren zur Durchführung einer isonaträmischen Dialyse sind zudem aus der DE 10 2017 116 097 A1 bekannt. Um während der Dialysebehandlung die Plasma-Natrium-Konzentration im Blut des Patienten möglichst nicht zu ändern wird diese vorab bestimmt oder abgeschätzt, indem zu Beginn der Dialysebehandlung die Dialysevorrichtung in einen Bypass-Modus geschaltet wird, bei dem der Dialysator nicht mehr von frischer Dialysierflüssigkeit durchströmt wird, das Blut des Patienten jedoch weiterhin den Dialysator durchströmt. Nach einer bestimmten Zeit korreliert die Natriumkonzentration der Dialysierflüssigkeit im Dialysator mit der Plasma-Natrium-Konzentration des Patienten und kann mittels einer geeigneten Messeinrichtung, wie einer Leitfähigkeitsmesszelle, erfasst werden.

Es zeigte sich jedoch, dass Leitfähigkeitsmessungen an der aus dem Dialysator ausströmenden Dialysierflüssigkeit, d.h. der verbrauchten Dialysierflüssigkeit, von Substanzen beeinflusst werden, die aus dem Blut in die Dialysierflüssigkeit übergegangen sind. Somit ist eine einfache Korrelation zwischen erfasstem Leitfähigkeitswert und der Natriumkonzentration nicht gegeben.

Natrium und die zugehörigen Anionen der entsprechenden Natriumsalze, insbesondere Chlorid und Hydrogencarbonat, liefern zwar den größten Beitrag zur Leitfähigkeit einer Dialysierflüssigkeit, jedoch wird die Leitfähigkeitsmessung von weiteren Stoffen beeinflusst, so dass eine einfache Umrechnung zwischen Leitfähigkeit und Natrium-Konzentration nicht mehr gegeben ist. Beispielsweise können erhöhte Kaliumkonzentrationen die Leitfähigkeit erhöhen. Es gibt aber auch Stoffe, die per se nicht leitfähig sind und dennoch die Leitfähigkeit beeinträchtigen können, da sie die Beweglichkeit der leitenden Ionen herabsetzen. Dieser Effekt wird von Toxinen und anderen harnpflichtigen Substanzen verursacht. Insbesondere zu Beginn einer Dialysebehandlung passieren viele dieser Stoffe die semipermeable Membran im Dialysator und gelangen so auf die Dialysatseite. Wird die Leitfähigkeit aufgrund dieser Substanzen herabgesetzt, so wird bei der Proportionierung frischer Dialysierflüssigkeit eine zu geringe Leitfähigkeit und damit eine zu geringe Natriumkonzentration eingestellt, so dass dem Patienten im Laufe der Dialysebehandlung unerwünscht Natrium entzogen wird. Auch eine starre Korrektur der Leitfähigkeit durch Addieren eines festen Betrags wäre nachteilig, da die Belastung mit Toxinen von Patient zu Patient und von Behandlung zu Behandlung unterschiedlich ausfallen kann.

Der Erfindung liegt somit die Aufgabe zugrunde, eine Vorrichtung zur extrakorporalen Blutbehandlung, insbesondere zur Hämodialyse bereitzustellen, womit die Dialysierflüssigkeit in ihrer Zusammensetzung automatisch und patientenindividuell angepasst werden kann, so dass unter anderem auch eine null-diffusive Natrium-Bilanzierung während der Dialyse realisiert werden kann. Eine weitere Aufgabe der vorliegenden Erfindung ist die Bereitstellung eines einfachen und dennoch genauen Verfahrens zur Bilanzregelung einer Dialysierflüssigkeit bei einer extrakorporalen Blutbehandlung, bei der lediglich Messwerte verwendet werden, die auf der Dialysatseite erfasst werden.

Die Aufgaben werden gelöst durch eine Vorrichtung zur extrakorporalen Blutbehandlung nach Anspruch 1, sowie ein Verfahren zur Bilanzregelung einer Dialysierflüssigkeit nach Anspruch 9.

Die erfindungsgemäße Vorrichtung zur extrakorporalen Blutbehandlung, insbesondere zur Hämodialyse, umfasst:

einen Dialysator, der einen Stoffaustausch zwischen einem Patientenblut und einer Dialysierflüssigkeit ermöglicht, eine Proportionierungseinheit, die frische Dialysierflüssigkeit bereitstellt und mit dem Dialysator über eine Zuführleitung zum Zuführen frischer Dialysierflüssigkeit verbunden ist, wobei der Dialysator ferner mit einer Abführleitung zum Ausleiten verbrauchter Dialysierflüssigkeit aus dem Dialysator verbunden ist,

eine erste Messeinrichtung, die in der Zuführleitung vorgesehen ist, zum Erfassen der Leitfähigkeit der frischen Dialysierflüssigkeit,

eine zweite Messeinrichtung, die in der Abführleitung vorgesehen ist, zum Erfassen der Leitfähigkeit der verbrauchten Dialysierflüssigkeit, und

eine Steuereinheit, die zum Datenaustausch mit der Proportionierungseinheit und den Messeinrichtungen verbunden ist,

dadurch gekennzeichnet, dass

in der Abführleitung eine dritte Messeinrichtung vorgesehen ist, zum Messen oder Abschätzen einer Beladung der verbrauchten Dialysierflüssigkeit mit harnpflichtigen Stoffen, und die dritte Messeinrichtung zum Datenaustauch mit der Steuereinheit verbunden ist und

die Steuereinheit ausgestaltet ist, auf Basis der während der Blutbehandlung kontinuierlich von der ersten Messeinrichtung und

der zweiten Messeinrichtung erfassten Leitfähigkeiten und der von der dritten Messeinrichtung erfassten oder abgeschätzten Beladung mit harnpflichtigen Stoffen die Konzentration eines Stoffes in der von der Proportionierungseinheit bereitgestellten frischen Dialysierflüssigkeit kontinuierlich während der Blutbehandlung so zu steuern oder regeln dass sie gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist.

Bei dem Dialysator der erfindungsgemäßen Vorrichtung handelt es sich vorzugsweise um einen handelsüblichen Dialysator, wie er für extrakorporale Blutbehandlungen eingesetzt wird. Er beinhaltet mehrere Hohlfaserkapillaren, die durch eine semipermeable Membran charakterisiert sind. Im Inneren des Dialysators durchströmt das Patientenblut die Kapillaren und werden die Kapillaren von außen von der Dialysierflüssigkeit umspült, welche die Toxine und weiteren Schadstoffe aus dem Blut des Patienten aufnimmt.

Die erfindungsgemäße Vorrichtung umfasst ferner eine Proportionierungseinheit in der die bei der Dialyse eingesetzte frische Dialysierflüssigkeit hergestellt wird. Vorzugsweise kommen dafür Prinzipien zum Einsatz, wie sie im Stand der Technik allgemein bekannt sind. Beispiele sind die eingangs erläuterten Varianten wie die leitfähigkeitsgesteuerte Proportionierung, die volumetrische Proportionierung oder eine Mischform aus beiden. Üblicherweise werden hierzu hochreines Wasser, eine basische erste Komponente und eine saure zweite Komponente miteinander vermischt.

Zur Unterscheidung der Dialysierflüssigkeiten wird in der vorliegenden Anmeldung die von der Proportionierungseinheit bereitgestellte und dem Dialysator zugeführte Dialysierflüssigkeit als "frische Dialysierflüssigkeit" bezeichnet und wird die aus dem Dialysator austretende Dialysierflüssigkeit als "verbrauchte Dialysierflüssigkeit" bezeichnet.

Die erfindungsgemäße Vorrichtung umfasst zudem eine Zuführleitung in der Form einer Fluidverbindung zwischen der Proportionierungseinheit und dem Dialysator, um die frische Dialysierflüssigkeit dem Dialysator zuzuführen. Der Dialysator ist ferner mit einer Abführleitung verbunden, die dem Ausleiten verbrauchter Dialysierflüssigkeit aus dem Dialysator dient und diese einem Abfluss zuleitet.

In der Zuführleitung ist eine erste Messeinrichtung vorgesehen zum Erfassen der Leitfähigkeit der frischen Dialysierflüssigkeit. Die erste Messeinrichtung ist vorzugsweise eine temperaturkompensierende Leitfähigkeitszelle, so dass eine temperaturkompensierte Leitfähigkeit der frischen Dialysierflüssigkeit erfasst werden kann.

In der Abführleitung ist eine zweite Messeinrichtung vorgesehen zum Erfassen der Leitfähigkeit der verbrauchten Dialysierflüssigkeit. Die zweite Messeinrichtung ist ebenfalls vorzugsweise eine temperaturkompensierende Leitfähigkeitszelle.

In der Abführleitung ist ferner eine dritte Messeinrichtung vorgesehen, die in Strömungsrichtung entweder vor oder nach der zweiten Messeinrichtung angeordnet ist. Die dritte Messeinrichtung ist dazu gedacht, die Beladung der verbrauchten Dialysierflüssigkeit mit harnpflichtigen Stoffen zu bestimmen oder abzuschätzen. Vorzugsweise handelt es sich bei der dritten Messeinrichtung um einen optischen Sensor, der die Absorptionseigenschaft der verbrauchten Dialysierflüssigkeit erfasst, wie beispielsweise die Extinktion. Vorzugsweise wird die Absorptionseigenschaft im ultravioletten Bereich zwischen 235 nm und 400 nm erfasst. Weiter bevorzugt wird die Absorptionseigenschaft von Licht mit einer Wellenlänge von $285\pm15$ nm erfasst. Alternativ ist auch ein enzymatischer oder ein anderer elektrochemischer Sensor denkbar.

Mit dem Begriffen "Beladung der verbrauchten Dialysierflüssigkeit mit harnpflichtigen Stoffen" und "Beladung mit harnpflichtigen Stoffen" wird die Summe aller Stoffe bezeichnet, die bei der Dialyse aus dem Blut des Patienten in die Dialysierflüssigkeit übergehen und somit in der aus dem Dialysator ausgeleiteten verbrauchten Dialysierflüssigkeit enthalten sind. In der Summe verändern diese Stoffe Eigenschaften der verbrauchten Dialysierflüssigkeit, wie beispielsweise die Absorptionseigenschaften, so dass mittels der dritten Messeinrichtung das Ausmaß der Beladung der verbrauchten Dialysierflüssigkeit mit harnpflichtigen Stoffen zumindest abgeschätzt werden kann.

Die erfindungsgemäße Vorrichtung umfasst ferner eine Steuereinheit, die zum Datenaustausch mit der Proportionierungseinheit und den drei Messeinrichtungen verbunden ist. Die Verbindung kann beispielsweise über geeignete elektrische Leitungen und/oder drahtlos realisiert werden. Somit ist eine Übermittlung der von den Messeinrichtungen erfassten Werte an die Steuereinheit, eine Ansteuerung der Proportionierungseinheit und eine Zustandserfassung aller mit der Steuereinheit verbundenen Komponenten möglich.

Zudem ist die Steuereinheit derart ausgestaltet, dass sie auf Basis der von der ersten Messeinrichtung und der zweiten Messeinrichtung erfassten Leitfähigkeiten und der von der dritten Messeinrichtung erfassten oder abgeschätzten Beladung mit harnpflichtigen Stoffen die Konzentration eines Stoffes in der von der Proportionierungseinheit bereitgestellten frischen Dialysierflüssigkeit während der Blutbehandlung so zu steuern oder regeln, dass sie gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist.

Mit der erfindungsgemäßen Vorrichtung wird vorzugsweise eine Natrium-Bilanzregelung durchgeführt, wobei gemäß einer weiter bevorzugten Ausführungsform während der Dialyse auch eine null-diffusive Natrium-Bilanzierung mit hoher Genauigkeit realisierbar ist.

Gemäß einer bevorzugten Ausführungsform der erfindungsgemäßen Vorrichtung weist diese ferner auf:

ein erstes Ventil, das in der Zuführleitung vorgesehen ist,

ein zweites Ventil, das in der Abführleitung vorgesehen ist,

eine Bypassleitung, die die Zuführleitung stromaufwärts des ersten Ventils mit der Abführleitung stromabwärts des zweiten Ventils unter Umgehung des Dialysators verbindet, und

ein drittes Ventil, das in der Bypassleitung vorgesehen ist,

wobei die erste Messeinrichtung in der Zuführleitung stromaufwärts der Bypassleitung vorgesehen ist und die zweite Messeinrichtung und die dritte Messeinrichtung in der Abführleitung stromabwärts der Bypassleitung vorgesehen sind, und

die Ventile zum Datenaustauch mit der Steuereinheit verbunden sind.

In dieser Ausführungsform ist in der Zuführleitung ist ein erstes Ventil und in der Abführleitung ein zweites Ventil vorgesehen und ist zudem eine Bypassleitung als eine Fluidverbindung zwischen der Zuführleitung und der Abführleitung vorgesehen, um den Dialysator zu umgehen, wobei die Bypassleitung mit der Zuführleitung stromaufwärts des ersten Ventils verbunden ist und mit der Abführleitung stromabwärts des zweiten Ventils verbunden ist. In der Bypassleitung ist ferner ein drittes Ventil vorgesehen.

Die drei Ventile sind zum Datenaustauch mit der Steuereinheit verbunden, so dass beispielsweise eine Ansteuerung der Ventile und deren Zustandserfassung möglich ist.

Die Bypassleitung ist so angeordnet, dass die erste Messeinrichtung in der Zuführleitung stromaufwärts der Bypassleitung vorgesehen ist und die zweite Messeinrichtung und die dritte Messeinrichtung in der Abführleitung stromabwärts der Bypassleitung vorgesehen sind.

Mit dem ersten, zweiten und dritten Ventil können jeweils unabhängig voneinander die Fluiddurchflüsse in der Zuführleitung, der Abführleitung bzw. der Bypassleitung gesteuert und/oder geregelt werden.

Während der Blutbehandlung und somit auch während der Erfassung der Leitfähigkeiten mittels der ersten und zweiten Messeinrichtung und während der Erfassung oder Abschätzung der Beladung der verbrauchten Dialysierflüssigkeit mit harnpflichtigen Stoffen mittels der dritten Messeinrichtung werden die Ventile von der Steuereinheit so angesteuert, dass das erste und zweite Ventil geöffnet und das dritte Ventil in der Bypassleitung geschlossen ist.

Wie bereits oben angegeben, ist bei der erfindungsgemäßen Vorrichtung die Steuereinheit derart ausgestaltet, dass auf Basis der von der ersten Messeinrichtung und der zweiten Messeinrichtung erfassten Leitfähigkeiten und der von der dritten Messeinrichtung erfassten oder abgeschätzten Beladung mit harnpflichtigen Stoffen die Konzentration eines Stoffes in der von der Proportionierungseinheit bereitgestellten frischen Dialysierflüssigkeit so gesteuert oder geregelt werden kann, dass sie gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist.

Da, wie eingangs dargelegt, die Leitfähigkeitsmessung in der verbrauchten Dialysierflüssigkeit durch verschiedene darin enthaltene Stoffe, wie beispielsweise Toxine, derart beeinflusst werden kann, dass ein falscher Wert der Konzentration des zu bestimmenden Stoffes erhalten wird, wird bei der erfindungsgemäßen Vorrichtung mittels der dritten Messeinrichtung die Beladung der verbrauchten Dialysierflüssigkeit mit harnpflichtigen Stoffen gemessen oder abgeschätzt. Bei der Berechnung der Konzentration des Stoffes in der verbrauchten Dialysierflüssigkeit wird von der Steuereinheit der Grad der Beladung mit harnpflichtigen Stoffen berücksichtigt und damit eine genauere Konzentrationsbestimmung ermöglicht. Auf Basis dieses genaueren Konzentrationswertes ist die Proportionierungseinheit in der Lage, mit verbesserter Genauigkeit eine frische Dialysierflüssigkeit bereitzustellen, die beispielsweise dieselbe Konzentration des Stoffes enthält wie die verbrauchte Dialysierflüssigkeit. Somit kann beispielsweise eine genauere isonaträmische Dialyse sichergestellt werden. Alternativ kann, falls gewünscht, ein vorgegebener Konzentrationsunterschied des Stoffes zwischen frischer und verbrauchter Dialysierflüssigkeit eingestellt werden, so dass die Konzentration dieses Stoffes im Patientenblut gezielt und vor allem mit verbesserter Genauigkeit verändert werden kann.

Somit ist mit der erfindungsgemäßen Vorrichtung eine verbesserte Bilanzregelung der Dialysierflüssigkeit während der Dialyse möglich, so dass genauer festgelegt werden kann, ob ein in der Dialysierflüssigkeit gelöster Stoff oder wieviel davon beim Durchgang durch den Dialysator auf das Blut des Patienten übergeht. Es kann auch genauer festgelegt werden, ob ein im Patientenblut gelöster Stoff oder wieviel davon beim Durchgang durch den Dialysator in die Dialysierflüssigkeit übergeht. Damit ist eine schonendere Dialysebehandlung sichergestellt, da mittel- und langfristig hohe Ultrafiltrationsraten vermieden werden könnten, und sind schonendere Änderungen des Wasser- und Elektrolythaushalts des Patienten realisierbar.

Der Vorteil der erfindungsgemäßen Vorrichtung und des erfindungsgemäßen Verfahrens liegt unter anderem darin, dass während der Dialyse, d.h. kontinuierlich und ohne diese zu unterbrechen, die Konzentration eines Stoffes in der frischen Dialysierflüssigkeit gesteuert bzw. geregelt werden kann. Zu diesem Zweck werden während der Dialyse mittels der drei Messeinrichtungen kontinuierlich Messwerte erfasst und an die Steuereinheit übermittelt und legt die Steuereinheit anhand der erhaltenen Messwerte kontinuierlich die Zusammensetzung der frischen Dialysierflüssigkeit fest.

Zudem sind keine zusätzlichen Verfahrensschritte zur Bestimmung der Konzentration des Stoffes im Patientenblut notwendig, um die Proportionierung der frischen Dialysierflüssigkeit entsprechend darauf einzustellen. So ist eine patientenindividuelle Dialyse auch ohne eine Entnahme und Analyse des Patientenblutes möglich. Auch der wie in der DE 10 2017 116 097 A1 notwendige Bypass-Modus zur Bestimmung oder Abschätzung der Natriumkonzentration im Patientenblut kann bei der erfindungsgemäßen Vorrichtung entfallen. Auch ist es nicht notwendig, dass während der Dialyse diese unterbrochen werden muss, wenn die Konzentration des Stoffes erneut bestimmt werden soll.

Gemäß einer weiteren bevorzugten Ausführungsform ist die Steuereinheit derart ausgestaltet, dass anhand der gemessenen oder abgeschätzten Beladung der verbrauchten Dialysierflüssigkeit mit harnpflichtigen Stoffen und der erfassten Leitfähigkeit der verbrauchten Dialysierflüssigkeit eine korrigierte Leitfähigkeit der verbrauchten Dialysierflüssigkeit berechnet werden kann, und anhand der korrigierten Leitfähigkeit der verbrauchten Dialysierflüssigkeit die Zusammensetzung der frischen Dialysierflüssigkeit so festgelegt werden kann, dass die Leitfähigkeit der frischen Dialysierflüssigkeit gleich der korrigierten Leitfähigkeit der verbrauchten Dialysierflüssigkeit ist. Damit kann mit der erfindungsgemäßen Vorrichtung beispielsweise eine isonaträmische Dialyse mit verbesserter Genauigkeit durchgeführt werden.

Die Korrektur der erfassten Leitfähigkeit der verbrauchten Dialysierflüssigkeit kann beispielsweise durchgeführt werden, indem die von der zweiten Messvorrichtung gemessene Leitfähigkeit $C_{DO}$ und die von der dritten Messvorrichtung gemessene Extinktion A mathematisch miteinander kombiniert werden, um die korrigierte Leitfähigkeit $C_{DO,corr}$ zu erhalten:

$$C_{DO,corr} = f(C_{DO}, A)$$

Im einfachsten Fall bietet sich eine lineare Gleichung an. Aber auch Polynome höheren Grades oder eine Gleichung, die mithilfe eines künstlichen neuronalen Netzes ermittelt wurde, sind denkbar. Eine alternative Möglichkeit ist die Verwendung einer Lookup-Tabelle.

Soll beispielsweise eine null-diffusive Natrium-bilanzierte Dialyse durchgeführt werden, so wird die Dialysierflüssigkeit von der Proportionierungseinheit so zusammengemischt, dass ihre Leitfähigkeit $C_{DI}$ der korrigierten Leitfähigkeit der verbrauchten Dialysierflüssigkeit $C_{DO,corr}$ entspricht:

$$C_{DI} = C_{DO,corr}$$

Sind beide Leitfähigkeiten gleich, so findet im Dialysator kein diffusiver Austausch von Natriumionen zwischen Blutseite und Dialysatseite statt. Die Leitfähigkeit $C_{DI}$ bzw. $C_{DO,corr}$ entspricht somit auch der Plasmawasserleitfähigkeit $C_{BI}$, die proportional zur Plasma-Natrium-Konzentration im Patientenblut ist. Es ist daher auch möglich, nach anfänglicher Ermittlung der Plasma-Natrium-Konzentration die Dialysierflüssigkeit so einzustellen, dass dem Patienten eine definierte Menge Natrium entzogen oder verabreicht werden kann. Der Natriumfluss $\Delta J$ berechnet sich dabei zu:

$$\Delta J = k \cdot Q_D \cdot (C_{DI} - C_{DO,corr}) + k \cdot Q_{UF} \cdot C_{DO,corr}$$

mit dem Dialysierflüssigkeitsfluss $Q_D$, der Ultrafiltrationsrate $Q_{UF}$ und einem Faktor $k$, mit welchem die Leitfähigkeit in eine Natriumkonzentration umgerechnet wird, wobei auch hier wieder komplexere Umrechnungen zum Einsatz kommen können.

Darüber hinaus wird mit der vorliegenden Erfindung ermöglicht, die absolut entzogene Menge an Natrium zu bestimmen. Entweder anhand vorheriger Gleichung unter Berücksichtigung der Therapiedauer oder: Bei bekanntem Ultrafiltrationsvolumen und der Kenntnis der Plasma-Natrium-Konzentration im Verlauf der Dialysebehandlung entspricht die entzogene Menge an Natrium dem Produkt aus Ultrafiltrationsvolumen und Plasma-Natrium-Konzentration.

Werden bei der erfindungsgemäßen Vorrichtung das erste Ventil und das zweite Ventil geschlossen und das dritte Ventil geöffnet, befindet sich die Vorrichtung im Bypass. Die Dialysierflüssigkeit strömt dann unbeeinflusst vom Dialysator durch das dritte Ventil, vorbei an der zweiten und der dritten Messvorrichtung und in den Abfluss. In diesem Zustand fließt durch die erste und die zweite Messeinrichtung dieselbe Flüssigkeit, so dass der Zustand des Bypasses genutzt werden kann, die Funktionsweise beider Sensoren zu vergleichen und mögliche Differenzen durch einen Abgleich auf null zu setzen. Des Weiteren kann der Zustand des Bypasses genutzt werden, die dritte Messvorrichtung zu kalibrieren, da für eine Kalibrierung frische Dialysierflüssigkeit benötigt wird.

Die erfindungsgemäße Vorrichtung kann zudem eine Bilanzierungseinheit umfassen, die ausgestaltet ist, zu bestimmen, wieviel Wasser dem Patientenblut während der Blutbehandlung entzogen wird. Die Bilanzierungseinheit kann beispielsweise durch Vergleich und gegebenenfalls Anpassen der Durchflussmenge der Dialysierflüssigkeit sicherstellen, dass dem Patienten genau die vorgeschriebene Menge an überschüssigem Wasser entzogen wird.

Bei dem nicht beanspruchten Verfahren zur extrakorporalen Blutbehandlung, insbesondere zur Hämodialyse, mittels eines Dialysators, der einen Stoffaustausch zwischen einem Patientenblut und einer Dialysierflüssigkeit ermöglicht, werden während der Blutbehandlung die Leitfähigkeiten einer dem Dialysator zugeführten frischen Dialysierflüssigkeit und einer aus dem Dialysator ausgeleiteten verbrauchten Dialysierflüssigkeit erfasst, und wird eine Beladung der verbrauchten Dialysierflüssigkeit mit harnpflichtigen Stoffen gemessen oder abgeschätzt. Ferner legt eine Steuereinheit anhand der Daten der Leitfähigkeiten und der Beladung mit harnpflichtigen Stoffen die Zusammensetzung der frischen Dialysierflüssigkeit so fest, dass die Konzentration eines Stoffes in der frischen Dialysierflüssigkeit gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist, und stellt eine Proportionierungseinheit eine frische Dialysierflüssigkeit mit der von der Steuereinheit festgelegten Zusammensetzung bereit zum Zuführen zu dem Dialysator.

Wenn gemäß einer bevorzugten Ausführungsform des Verfahrens die Zusammensetzung der frischen Dialysierflüssigkeit so festlegt werden soll, dass die Konzentration des Stoffes in der frischen Dialysierflüssigkeit gleich der Konzentration des Stoffes in der verbrauchten Dialysierflüssigkeit ist, wie beispielsweise bei einer isonaträmischen Dialyse, berechnet die Steuereinheit anhand der gemessenen oder abgeschätzten Beladung der verbrauchten Dialysierflüssigkeit mit harnpflichtigen Stoffen und der erfassten Leitfähigkeit der verbrauchten Dialysierflüssigkeit eine korrigierte Leitfähigkeit der verbrauchten Dialysierflüssigkeit und legt die Zusammensetzung der frischen Dialysierflüssigkeit so fest, dass die Leitfähigkeit der frischen Dialysierflüssigkeit gleich der korrigierten Leitfähigkeit der verbrauchten Dialysierflüssigkeit ist.

Bei dem Verfahren zur extrakorporalen Blutbehandlung ist die Konzentration des Stoffes in der Dialysierflüssigkeit vorzugsweise eine Natriumkonzentration. Somit ist während der Dialyse eine genauere Natrium-Bilanzierung, vorzugsweise eine null-diffusive Natrium-Bilanzierung, realisierbar.

Mit dem Verfahren zur extrakorporalen Blutbehandlung werden dieselben Vorteile erzielt, wie sie oben bei der Erläuterung der erfindungsgemäßen Vorrichtung zur extrakorporalen Blutbehandlung genannt wurden.

Gemäß der vorliegenden Erfindung wird ferner ein Verfahren zur Bilanzregelung bzw. Überwachung einer Dialysierflüssigkeit bei einer extrakorporalen Blutbehandlung, insbesondere einer Hämodialyse, bei der die Dialysierflüssigkeit einen Dialysator durchströmt, bereitgestellt. Bei diesem Verfahren werden die Leitfähigkeiten einer dem Dialysator zugeführten frischen Dialysierflüssigkeit und einer aus dem Dialysator ausgeleiteten verbrauchten Dialysierflüssigkeit während der Blutbehandlung kontinuierlich erfasst, und wird eine Beladung der verbrauchten Dialysierflüssigkeit mit harnpflichtigen Stoffen gemessen oder abgeschätzt. Zudem wird anhand der Daten der Leitfähigkeiten und der Beladung mit harnpflichtigen Stoffen mittels einer Steuereinheit die Zusammensetzung der frischen Dialysierflüssigkeit während der Blutbehandlung kontinuierlich so festlegt, dass die Konzentration eines Stoffes in der frischen Dialysierflüssigkeit gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist, und stellt anhand der von der Steuereinheit festgelegten Zusammensetzung eine Proportionierungseinheit eine frische Dialysierflüssigkeit mit dieser Zusammensetzung bereit zum Zuführen zu dem Dialysator.

Wenn für die durchzuführende Dialyse die Zusammensetzung der frischen Dialysierflüssigkeit so festlegt wird, dass die Konzentration des Stoffes in der frischen Dialysierflüssigkeit gleich der Konzentration des Stoffes in der verbrauchten Dialysierflüssigkeit ist, wie beispielsweise bei einer isonaträmischen Dialyse, berechnet gemäß einer bevorzugten Ausführungsform des Verfahrens zur Bilanzregelung einer Dialysierflüssigkeit die Steuereinheit anhand der gemessenen oder abgeschätzten Beladung der verbrauchten Dialysierflüssigkeit mit harnpflichtigen Stoffen und der erfassten Leitfähigkeit der verbrauchten Dialysierflüssigkeit eine korrigierte Leitfähigkeit der verbrauchten Dialysierflüssigkeit und legt die Zusammensetzung der frischen Dialysierflüssigkeit so fest, dass die Leitfähigkeit der frischen Dialysierflüssigkeit gleich der korrigierten Leitfähigkeit der verbrauchten Dialysierflüssigkeit ist.

Bei dem erfindungsgemäßen Verfahren zur Bilanzregelung bzw. Überwachung einer Dialysierflüssigkeit bei einer extrakorporalen Blutbehandlung ist die Konzentration des Stoffes in der Dialysierflüssigkeit vorzugsweise eine Natriumkonzentration. Somit ist während der Dialyse eine genauere Natrium-Bilanzierung, vorzugsweise eine nulldiffusive Natrium-Bilanzierung, realisierbar.

Mit dem erfindungsgemäßen Verfahren zur Bilanzregelung bzw. Überwachung einer Dialysierflüssigkeit bei einer extrakorporalen Blutbehandlung werden dieselben Vorteile erzielt, wie sie oben bei der Erläuterung der erfindungsgemäßen Vorrichtung zur extrakorporalen Blutbehandlung genannt wurden.

Das oben erläuterte Verfahren zur extrakorporalen Blutbehandlung wie auch das Verfahren zur Bilanzregelung einer Dialysierflüssigkeit bei einer extrakorporalen Blutbehandlung wird vorzugsweise unter Verwendung der erfindungsgemäßen Vorrichtung zur extrakorporalen Blutbehandlung durchgeführt.

Bestandteil beider Verfahren kann zudem, entsprechend einer bevorzugten Ausführungsform, die Durchführung eines Bypass-Modus sein, bei dem das erste Ventil und das zweite Ventil geschlossen und das dritte Ventil geöffnet werden. In diesem Zustand fließt durch die erste und die zweite Messeinrichtung dieselbe Flüssigkeit, so dass der Zustand des Bypasses genutzt werden kann, die Funktionsweise beider Sensoren zu vergleichen und mögliche Differenzen durch einen Abgleich auf null zu setzen. Des Weiteren kann der Zustand des Bypasses genutzt werden, die dritte Messvorrichtung zu kalibrieren, da für eine Kalibrierung frische Dialysierflüssigkeit benötigt wird.

Sowohl bei der erfindungsgemäßen Vorrichtung wie auch dem erfindungsgemäßen Verfahren können gemäß bevorzugter Ausführungsformen Daten der Messeinrichtungen und/oder Charakteristika der von der Proportionierungseinheit angemischten Dialysierflüssigkeit auf einem Bildschirm oder einem Datenmanagementsystem angezeigt werden, um die Abläufe für das medizinische Personal und/oder den Anwender transparent zu gestalten. Dies betrifft insbesondere Leitfähigkeiten, Konzentrationen, Extinktionen, pH-Werte, Temperaturen und/oder Drücke. Gemäß einer weiteren Ausführungsform kann vorgesehen sein, dass bestimmte Daten nur als Empfehlung zur Verfügung gestellt werden. So kann das medizinische Personal basierend auf diesen empfohlenen Daten selbst entscheiden, ob der Empfehlung nachgegangen werden soll oder nicht, d.h. eine hypo-, iso- oder hypernaträmische Dialyse findet gemäß dieser Ausführungsform demnach nicht automatisch statt.

Gemäß bevorzugter Ausführungsformen der Vorrichtung bzw. des Verfahrens können erhobene Daten auf einer Patientenkarte, in der Dialysemaschine und/oder in einem Datenmanagementsystem abgespeichert werden. In den vergangenen Jahren durchgeführte Studien haben ergeben, dass die Plasma-Natrium-Konzentration eines Patienten im Vergleich zu anderen Parametern relativ konstant ist (Setpoint-Theorie):

- Peixoto, A. J.; Gowda, N.; Parikh, C. R., Santos, S. F. F.: "Long-Term Stability of Serum Sodium in Hemodialysis Patients", Blood Purif, 2010, 29, 264-267,

- Keen, M. L., Gotch, F. A.: "The association of the sodium setpoint to interdialytic weight gain and blood pressure in hemodialysis patients", The International Journal of Artificial Organs, 2007, 30, 971-979, und

-  Hecking, M.; Kainz, A.; Hörl, W. H.; Herkner, H., Sunder-Plassmann, G.: "Sodium Setpoint and Sodium Gradient: Influence on Plasma Sodium Change and Weight Gain", Am J Nephrol, 2011, 33, 39-48 .

So können die gespeicherten Daten, insbesondere die Leitfähigkeiten $C_{DI}$ und die korrigierte Leitfähigkeit $C_{DO,corr}$ sowie die daraus ermittelte Differenz und die Extinktion A mit Mitteln der deskriptiven Statistik (z.B. Mittelwert, Standardabweichung, zeitliche Korrelationen) ausgewertet werden, um eventuelle Veränderungen trotzdem zu erkennen, was auf eine allgemeine Änderung des gesundheitlichen Zustands hindeuten könnte.

Kurzbeschreibung der Figuren

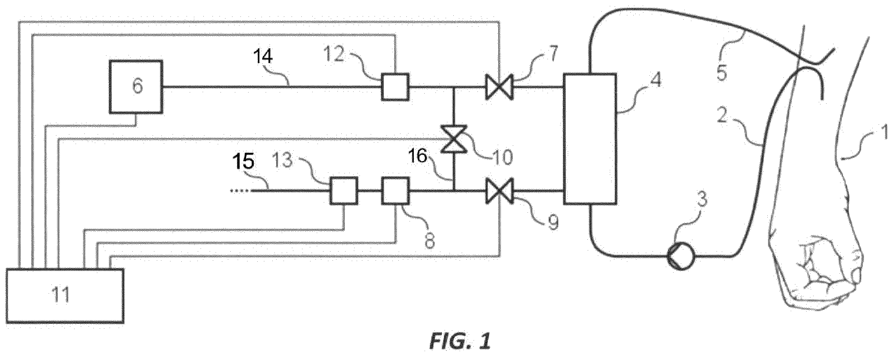

Fig. 1 ist eine schematische Darstellung einer Ausführungsform der erfindungsgemäßen Vorrichtung zur extrakorporalen Blutbehandlung.

Beschreibung der Ausführungsbeispiele

Eine schematische Umsetzung der erfindungsgemäßen Vorrichtung ist in FIG. 1 dargestellt. Einem Dialysepatienten 1 wird über das arterielle Schlauchsystem 2 mithilfe einer Fördereinrichtung 3 Blut entzogen. Das Blut gelangt in einen Dialysator 4, wo es von Toxinen und überschüssigem Wasser mittels Diffusion und/oder Ultrafiltration befreit wird. Anschließend wird es zu dem Patienten über das venöse Schlauchsystem 5 rückgeführt. Die Entnahme und Rückgabe über eine gemeinsame Kanüle ist ebenfalls denkbar.

Bei dem Dialysator 4 handelt es sich um einen wie oben beschriebenen handelsüblichen Dialysator, wie er für extrakorporale Blutbehandlungen eingesetzt wird. Die Dialysierflüssigkeit wird in der Proportionierungseinheit 6 hergestellt. Die erste Messeinrichtung 12, die in der Zuführleitung 14 vorgesehen ist, misst eine temperaturkompensierte Leitfähigkeit der frischen Dialysierflüssigkeit.

Im Hauptschluss, d.h. wenn die Dialysierflüssigkeit durch den Dialysator 4 strömt, ist das dritte Ventil 10, das in einer Bypassleitung 16 vorgesehen ist, die die Zuführleitung 14 stromaufwärts des ersten Ventils 7 mit der Abführleitung 15 stromabwärts des zweiten Ventils 9 unter Umgehung des Dialysators 4 verbindet, geschlossen. Die frische Dialysierflüssigkeit strömt somit durch die Zuführleitung 14 mit der ersten Messeinrichtung 12 und durch das erste Ventil 7 in den Dialysator 4, nimmt dort die Schadstoffe aus dem Blut auf und gibt gegebenenfalls andere Stoffe, insbesondere Hydrogencarbonat und/oder andere Elektrolyte bzw. Nichtelektrolyte, an das Blut ab. Nach Durchlauf durch den Dialysator 4 passiert die verbrauchte Dialysierflüssigkeit das zweite Ventil 9 sowie die zweite Messeinrichtung 8 und die dritte Messeinrichtung 13 in der Abführleitung 15. Bei der Messeinrichtung 8 handelt es sich um die gleiche Messeinrichtung wie erste Messeinrichtung 12. Die dritte Messvorrichtung 13 ist vorgesehen, die Beladung der verbrauchten Dialysierflüssigkeit mit harnpflichtigen Stoffen zu bestimmen oder abzuschätzen. In der vorliegenden Ausführungsform handelt es sich um einen optischen Sensor, der die Absorptionseigenschaft der verbrauchten Dialysierflüssigkeit im ultravioletten Bereich zwischen 235 nm und 400 nm misst. Vorzugsweise wird die Absorptionseigenschaft von Licht mit einer Wel-lenlänge von $285 \pm 15$ nm gemessen. Alternativ ist auch ein enzymatischer oder ein anderer elektrochemischer Sensor denkbar.

Die Steuereinheit 11 erfasst die Messwerte und Zustände der Messeinrichtungen 8, 12 und 13, der Proportionierungseinheit 6 sowie der Ventile 7, 9 und 10. Des Weiteren gibt sie Befehle an die Proportionierungseinheit 6 und an die Ventile 7, 9 und 10 aus.

**Patentansprüche**

1. Vorrichtung zur extrakorporalen Blutbehandlung, insbesondere zur Hämodialyse, aufweisend:

einen Dialysator (4), der einen Stoffaustausch zwischen einem Patientenblut und einer Dialysierflüssigkeit ermöglicht,
eine Proportionierungseinheit (6), die frische Dialysierflüssigkeit bereitstellt und mit dem Dialysator (4) über eine Zuführleitung (14) zum Zuführen frischer Dialysierflüssigkeit verbunden ist, wobei der Dialysator (4) ferner mit einer Abführleitung (15) zum Ausleiten verbrauchter Dialysierflüssigkeit aus dem Dialysator (4) verbunden ist,
eine erste Messeinrichtung (12), die in der Zuführleitung (14) vorgesehen ist,
zum Erfassen der Leitfähigkeit der frischen Dialysierflüssigkeit,
eine zweite Messeinrichtung (8), die in der Abführleitung (15) vorgesehen ist,
zum Erfassen der Leitfähigkeit der verbrauchten Dialysierflüssigkeit,
eine Steuereinheit (11), die zum Datenaustausch mit der Proportionierungseinheit (6) und den Messeinrichtungen (8, 12) verbunden ist, und wobei in der Abführleitung (15) eine dritte Messeinrichtung (13) vorgesehen ist, zum Messen oder Abschätzen einer Beladung der verbrauchten Dialysierflüssigkeit mit harnpflichtigen Stoffen, und die dritte Messeinrichtung (13) zum Datenaustauch mit der Steuereinheit (11) verbunden ist,
**dadurch gekennzeichnet, dass**
die Steuereinheit (11) ausgestaltet ist, auf Basis der während der Blutbehandlung kontinuierlich von der ersten Messeinrichtung (12) und der zweiten Messeinrichtung (8) erfassten Leitfähigkeiten und der von der dritten Messeinrichtung (13) erfassten oder abgeschätzten Beladung mit harnpflichtigen Stoffen die Konzentration eines Stoffes in der von der Proportionierungseinheit (6) bereitgestellten frischen Dialysierflüssigkeit kontinuierlich während der Blutbehandlung so zu steuern oder regeln, dass sie gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist.

2. Vorrichtung nach Anspruch 1, die ferner aufweist:

ein erstes Ventil (7), das in der Zuführleitung (14) vorgesehen ist,

ein zweites Ventil (9), das in der Abführleitung (15) vorgesehen ist,

eine Bypassleitung (16), die die Zuführleitung (14) stromaufwärts des ersten Ventils (7) mit der Abführleitung (15) stromabwärts des zweiten Ventils (9) unter Umgehung des Dialysators (4) verbindet, und

ein drittes Ventil (10), das in der Bypassleitung (16) vorgesehen ist,

wobei die erste Messeinrichtung (12) in der Zuführleitung (14) stromaufwärts der Bypassleitung (16) vorgesehen ist und die zweite Messeinrichtung (8) und die dritte Messeinrichtung (13) in der Abführleitung (15) stromabwärts der Bypassleitung (16) vorgesehen sind, und

die Ventile (7, 9, 10) zum Datenaustauch mit der Steuereinheit (11) verbunden sind.

3. Vorrichtung nach Anspruch 2, wobei die Steuereinheit (11) ausgestaltet ist, während der Blutbehandlung und während der Erfassung der Leitfähigkeiten mittels der ersten und zweiten Messeinrichtung (12, 8) und während der Erfassung oder Abschätzung der Beladung der verbrauchten Dialysierflüssigkeit mit harnpflichtigen Stoffen mittels der dritten Messeinrichtung (13) die Ventile so anzusteuern, dass das erste und zweite Ventil (7, 9) geöffnet und das dritte Ventil (10) in der Bypassleitung (16) geschlossen ist.

4. Vorrichtung nach einem der vorhergehenden Ansprüche, wobei die Steuereinheit (11) ausgestaltet ist,

anhand der gemessenen oder abgeschätzten Beladung der verbrauchten Dialysierflüssigkeit mit harnpflichtigen Stoffen und der erfassten Leitfähigkeit der verbrauchten Dialysierflüssigkeit eine korrigierte Leitfähigkeit der verbrauchten Dialysierflüssigkeit zu berechnen, und

anhand der korrigierten Leitfähigkeit der verbrauchten Dialysierflüssigkeit die Zusammensetzung der frischen Dialysierflüssigkeit so festzulegen, dass die Leitfähigkeit der frischen Dialysierflüssigkeit gleich der korrigierten Leitfähigkeit der verbrauchten Dialysierflüssigkeit ist.

5. Vorrichtung nach einem der vorhergehenden Ansprüche, wobei die erste Messeinrichtung (12) und die zweite Messeinrichtung (8) temperaturkompensierte Leitfähigkeiten erfassen.

6. Vorrichtung nach einem der vorhergehenden Ansprüche, wobei die dritte Messeinrichtung (13) ein optischer Sensor, ein enzymatischer Sensor oder ein anderer elektrochemischer Sensor ist.

7. Vorrichtung nach Anspruch 6, wobei die dritte Messeinrichtung (13) ein optischer Sensor ist, der die Lichtabsorption im Wellenlängenbereich von 235 nm bis 400 nm erfasst.

8. Vorrichtung nach einem der vorhergehenden Ansprüche, wobei die Vorrichtung ferner eine Bilanzierungseinheit umfasst, die ausgestaltet ist, zu bestimmen, wieviel Wasser dem Patientenblut während der Blutbehandlung entzogen wird.

9. Verfahren zur Bilanzregelung oder Überwachung einer Dialysierflüssigkeit bei einer extrakorporalen Blutbehandlung, insbesondere einer Hämodialyse, bei der die Dialysierflüssigkeit einen Dialysator (4) durchströmt, wobei

während der Blutbehandlung die Leitfähigkeiten einer dem Dialysator (4) zugeführten frischen Dialysierflüssigkeit und einer aus dem Dialysator (4) ausgeleiteten verbrauchten Dialysierflüssigkeit kontinuierlich erfasst werden, und eine Beladung der verbrauchten Dialysierflüssigkeit mit harnpflichtigen Stoffen gemessen oder abgeschätzt wird,

anhand der Daten der Leitfähigkeiten und der Beladung mit harnpflichtigen Stoffen mittels einer Steuereinheit (11) die Zusammensetzung der frischen Dialysierflüssigkeit während der Blutbehandlung kontinuierlich so festgelegt wird, dass die Konzentration eines Stoffes in der frischen Dialysierflüssigkeit gleich der Konzentration des Stoffes in der verbrauchten Dialysierflüssigkeit ist, oder um einen vorgegebenen Wert größer oder kleiner als die Konzentration des Stoffes in der verbrauchten Dialysierflüssigkeit ist, und

anhand der von der Steuereinheit (11) festgelegten Zusammensetzung eine Proportionierungseinheit (6) eine frische Dialysierflüssigkeit mit dieser Zusammensetzung bereitstellt zum Zuführen zu dem Dialysator (4).

10. Verfahren nach Anspruch 9, wobei

wenn die Zusammensetzung der frischen Dialysierflüssigkeit so festgelegt wird, dass die Konzentration des Stoffes in der frischen Dialysierflüssigkeit gleich der Konzentration des Stoffes in der verbrauchten Dialysierflüssigkeit ist,

die Steuereinheit (11) anhand der gemessenen oder abgeschätzten Beladung der verbrauchten Dialysierflüssigkeit mit harnpflichtigen Stoffen und der erfassten Leitfähigkeit der verbrauchten Dialysierflüssigkeit eine korrigierte Leitfähigkeit der verbrauchten Dialysierflüssigkeit berechnet und die Zusammensetzung der frischen Dialysierflüssigkeit so festlegt, dass die Leitfähigkeit der frischen Dialysierflüssigkeit gleich der korrigierten Leitfähigkeit der verbrauchten Dialysierflüssigkeit ist.

11. Verfahren nach Anspruch 9 oder 10, wobei die Konzentration des Stoffes in der Dialysierflüssigkeit eine Natriumkonzentration ist.

12. Verfahren nach einem der Ansprüche 9 bis 11, wobei das Verfahren unter Verwendung der Vorrichtung gemäß einem der Ansprüche 1 bis 7 durchgeführt wird.

**Claims**

1. A device for extracorporeal blood treatment, in particular for hemodialysis, comprising:

   a dialyzer (4) rendering possible an exchange of substances between a patient's blood and a dialysis fluid,
   a proportioning unit (6), which provides fresh dialysis fluid and is connected to the dialyzer (4) via a supply line (14) for supplying fresh dialysis fluid, the dialyzer (4) further being connected to a discharge line (15) for discharging used dialysis fluid from the dialyzer (4),
   a first measuring apparatus (12) provided in the supply line (14) for detecting the conductivity of the fresh dialysis fluid,
   a second measuring apparatus (8), provided in the discharge line (15), for detecting the conductivity of the used dialysis fluid,
   a control unit (11) connected to the proportioning unit (6) and the measuring apparatuses (8, 12) for data exchange, and wherein
   a third measuring apparatus (13) is provided in the discharge line (15) for measuring or estimating a loading of the used dialysis fluid with urophanic substances, and the third measuring apparatus (13) is connected to the control unit (11) for data exchange,
   **characterized in that**
   the control unit (11) is designed to continuously control in open-loop or closed-loop fashion, on the basis of the conductivities detected continuously during blood treatment by the first measuring apparatus (12) and the second measuring apparatus (8), and the loading with urophanic substances detected or estimated by the third measuring apparatus (13), the concentration of a substance in the fresh dialysis fluid provided by the proportioning unit (6) during the blood treatment in such a way that it is equal to the concentration of the substance in the used dialysis fluid or is greater or less than the concentration of the substance in the used dialysis fluid by a predetermined value.

2. The device according to claim 1, further comprising:

   a first valve (7) provided in the supply line (14),
   a second valve (9) provided in the discharge line (15),
   a bypass line (16) connecting the supply line (14) upstream of the first valve (7) to the discharge line (15) downstream of the second valve (9), bypassing the dialyzer (4), and
   a third valve (10) provided in the bypass line (16),
   wherein the first measuring apparatus (12) is provided in the supply line (14) upstream of the bypass line (16), and the second measuring apparatus (8) and the third measuring apparatus (13) are provided in the discharge line (15) downstream of the bypass line (16), and
   the valves (7, 9, 10) are connected to the control unit (11) for data exchange.

3. The device according to claim 2, wherein the control unit (11) is designed to drive the valves during the blood treatment and during the detection of the conductivities by means of the first and second measuring apparatuses (12, 8), and during the detection or estimation of the loading of the used dialysis fluid with urophanic substances by means of the third measuring apparatus (13) in such a way that the first and second valves (7, 9) are open and the third valve (10) in the bypass line (16) is closed.

4. The device according to any of the preceding claims, wherein the control unit (11) is designed

to calculate a corrected conductivity of the used dialysis fluid on the basis of the measured or estimated loading of the used dialysis fluid with urophanic substances and the detected conductivity of the used dialysis fluid, and determine the composition of the fresh dialysis fluid on the basis of the corrected conductivity of the used dialysis fluid so that the conductivity of the fresh dialysis fluid is equal to the corrected conductivity of the used dialysis fluid.

5. The device according to any of the preceding claims, wherein the first measuring apparatus (12) and the second measuring apparatus (8) detect temperature-compensated conductivities.

6. The device according to any of the preceding claims, wherein the third measuring apparatus (13) is an optical sensor, an enzymatic sensor or another electrochemical sensor.

7. The device according to claim 6, wherein the third measuring apparatus (13) is an optical sensor that detects light absorption in the wavelength range from 235 nm to 400 nm.

8. The device according to any of the preceding claims, wherein the device further comprises a balancing unit designed to determine how much water is removed from the patient's blood during blood treatment.

9. A method for balance control or monitoring of a dialysis fluid in an extracorporeal blood treatment, in particular a hemodialysis, in which the dialysis fluid flows through a dialyzer (4), wherein,

during the blood treatment, the conductivities of a fresh dialysis fluid supplied to the dialyzer (4) and of a used dialysis fluid discharged from the dialyzer (4) are detected continuously, and a loading of the used dialysis fluid with urophanic substances is measured or estimated,
on the basis of the data of the conductivities and the loading with urophanic substances by means of a control unit (11), the composition of the fresh dialysis fluid during blood treatment is determined continuously in such a way that the concentration of a substance in the fresh dialysis fluid is equal to the concentration of the substance in the used dialysis fluid, or is greater or less than the concentration of the substance in the used dialysis fluid by a predetermined value, and
on the basis of the composition determined by the control unit (11), a proportioning unit (6) provides a fresh dialysis fluid with this composition for supply to the dialyzer (4).

10. The method according to claim 9, wherein,

when the composition of the fresh dialysis fluid is determined such that the concentration of the substance in the fresh dialysis fluid is equal to the concentration of the substance in the used dialysis fluid,
the control unit (11) calculates on the basis of the measured or estimated loading of the used dialysis fluid with urophanic substances and the detected conductivity of the used dialysis fluid, a corrected conductivity of the used dialysis fluid and determines the composition of the fresh dialysis fluid in such a way that the conductivity of the fresh dialysis fluid is equal to the corrected conductivity of the used dialysis fluid.

11. The method according to claim 9 or 10, wherein the concentration of the substance in the dialysis fluid is a sodium concentration.

12. The method according to any of claims 9 to 11, wherein the method is carried out using the device according to any of claims 1 to 7.

**Revendications**

1. Dispositif de traitement extracorporel du sang, en particulier pour l'hémodialyse, présentant :

un dialyseur (4) qui permet un échange de substances entre un sang de patient et un liquide de dialyse,
une unité de dosage (6) qui fournit du liquide de dialyse frais et est connectée au dialyseur (4) par l'intermédiaire d'une conduite d'alimentation (14) pour l'alimentation en liquide de dialyse frais, dans lequel le dialyseur (4) est en outre connecté à une conduite d'évacuation (15) pour l'évacuation de liquide de dialyse usé du dialyseur (4),
un premier appareil de mesure (12) qui est prévu dans la conduite d'alimentation (14) pour détecter la conductivité du liquide de dialyse frais,
un deuxième appareil de mesure (8) qui est prévu dans la conduite d'évacuation (15) pour détecter la conductivité du liquide de dialyse usé,

un dispositif de commande (11) qui est connecté à l'unité de dosage (6) et aux appareils de mesure (8, 12) pour l'échange de données, et dans lequel un troisième appareil de mesure (13) est prévu dans la conduite d'évacuation (15) pour la mesure ou l'estimation d'une charge du liquide de dialyse usé avec des substances urinaires, et le troisième appareil de mesure (13) est connecté au dispositif de commande (11) pour l'échange de données,

**caractérisé en ce que**

le dispositif de commande (11) est conçu pour, sur la base des conductivités détectées en continu pendant le traitement du sang par le premier appareil de mesure (12) et le deuxième appareil de mesure (8) et de la charge en substances urinaires détectée ou estimée par le troisième appareil de mesure (13), commander ou réguler en continu pendant le traitement du sang la concentration d'une substance dans le liquide de dialyse frais fourni par l'unité de dosage (6), de sorte qu'elle soit égale à la concentration de la substance dans le liquide de dialyse usé ou qu'elle soit supérieure ou inférieure d'une valeur prédéterminée à la concentration de la substance dans le liquide de dialyse usé.

2. Dispositif selon la revendication 1, qui présente en outre :

une première vanne (7) qui est prévue dans la conduite d'alimentation (14),
une deuxième vanne (9) qui est prévue dans la conduite d'évacuation (15),
une conduite de dérivation (16) qui connecte la conduite d'alimentation (14) en amont de la première vanne (7) à la conduite d'évacuation (15) en aval de la deuxième vanne (9) en contournant le dialyseur (4), et
une troisième vanne (10) qui est prévue dans la conduite de dérivation (16),
dans lequel le premier appareil de mesure (12) est prévu dans la conduite d'alimentation (14) en amont de la conduite de dérivation (16), et le deuxième appareil de mesure (8) et le troisième appareil de mesure (13) sont prévus dans la conduite d'évacuation (15) en aval de la conduite de dérivation (16), et
les vannes (7, 9, 10) sont connectées au dispositif de commande (11) pour l'échange de données.

3. Dispositif selon la revendication 2, dans lequel le dispositif de commande (11) est conçu pour, pendant le traitement du sang et pendant la détection des conductivités au moyen des premier et du deuxième appareils de mesure (12, 8) et pendant la détection ou l'estimation de la charge du liquide de dialyse usé en substances urinaires au moyen du troisième appareil de mesure (13), commander les vannes de sorte que les première et deuxième vannes (7, 9) soient ouvertes et la troisième vanne (10) dans la conduite de dérivation (16) soit fermée.

4. Dispositif selon l'une quelconque des revendications précédentes, dans lequel le dispositif de commande (11) est conçu pour

calculer, à l'aide de la charge mesurée ou estimée du liquide de dialyse usé en substances urinaires et de la conductivité détectée du liquide de dialyse usé, une conductivité corrigée du liquide de dialyse usé, et
à l'aide de la conductivité corrigée du liquide de dialyse usé, déterminer la composition du liquide de dialyse frais, de sorte que la conductivité du liquide de dialyse frais soit égale à la conductivité corrigée du liquide de dialyse usé.

5. Dispositif selon l'une quelconque des revendications précédentes, dans lequel le premier appareil de mesure (12) et le deuxième appareil de mesure (8) détectent des conductivités compensées en température.

6. Dispositif selon l'une quelconque des revendications précédentes, dans lequel le troisième appareil de mesure (13) est un capteur optique, un capteur enzymatique ou un autre capteur électrochimique.

7. Dispositif selon la revendication 6, dans lequel le troisième appareil de mesure (13) est un capteur optique qui détecte l'absorption de lumière dans la gamme de longueurs d'onde de 235 nm à 400 nm.

8. Dispositif selon l'une quelconque des revendications précédentes, dans lequel le dispositif comprend en outre une unité de bilan qui est conçue pour déterminer la quantité d'eau qui est extraite du sang de patient pendant le traitement du sang.

9. Procédé de régulation de bilan ou de surveillance d'un liquide de dialyse lors d'un traitement extracorporel du sang, en particulier d'une hémodialyse lors de laquelle le liquide de dialyse traverse un dialyseur (4), dans lequel

pendant le traitement du sang, les conductivités d'un liquide de dialyse frais alimenté au dialyseur (4) et d'un liquide de dialyse usé évacué du dialyseur (4) sont détectées en continu, et une charge du liquide de dialyse usé en substances urinaires est mesurée ou estimée,

à l'aide des données des conductivités et de la charge en substances urinaires, la composition du liquide de dialyse frais pendant le traitement du sang est déterminée en continu au moyen d'un dispositif de commande (11), de sorte que la concentration d'une substance dans le liquide de dialyse frais soit égale à la concentration de la substance dans le liquide de dialyse usé ou soit supérieure ou inférieure d'une valeur prédéterminée à la concentration de la substance dans le liquide de dialyse usé, et

à l'aide de la composition déterminée par le dispositif de commande (11), une unité de dosage (6) fournit un liquide de dialyse frais avec cette composition pour l'alimenter au dialyseur (4).

10. Procédé selon la revendication 9, dans lequel

lorsque la composition du liquide de dialyse frais est déterminée de sorte que la concentration de la substance dans le liquide de dialyse frais soit égale à la concentration de la substance dans le liquide de dialyse usé,

le dispositif de commande (11) calcule une conductivité corrigée du liquide de dialyse usé à l'aide de la charge mesurée ou estimée du liquide de dialyse usé en substances urinaires et de la conductivité détectée du liquide de dialyse usé et détermine la composition du liquide de dialyse frais de sorte que la conductivité du liquide de dialyse frais soit égale à la conductivité corrigée du liquide de dialyse usé.

11. Procédé selon la revendication 9 ou 10, dans lequel la concentration de la substance dans le liquide de dialyse est une concentration en sodium.

12. Procédé selon l'une quelconque des revendications 9 à 11, dans lequel le procédé est mis en oeuvre en utilisant le dispositif selon l'une quelconque des revendications 1 à 7.

FIG. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0097366 A2
- WO 2010112223 A1
- DE 69916053 T2
- DE 102017116097 A1

### In der Beschreibung aufgeführte Nicht-Patentliteratur

- **FLYTHE, J. E. ; KIMMEL, S. E ; BRUNELLI, S. M.** Rapid fluid removal during dialysis is associated with cardiovascular morbidity and mortality. *Kidney International,* 2010, vol. 79, 250-257
- **SARAN, R ; BRAGG-GRESHAM, J. ; LEVIN, N ; TWARDOWSKI, Z ; WIZEMANN, V ; SAITO, A ; KIMATA, N ; GILLESPIE, B.** Longer treatment time and slower ultrafiltration in hemodialysis: Associations with reduced mortality in the DOPPS. *Kidney International,* 2006, vol. 69, 1222-1228
- **DE PAULA, F. M. ; PEIXOTO, A. J ; PINTO, L. V. ; DORIGO, D. ; PATRICIO, P. J. M ; SANTOS, S. F. F.** Clinical consequences of an individualized dialysate sodium Prescription in hemodialysis patients. *Kidney International,* 2004, vol. 66, 1232-1238
- **BASILE, C ; LOMONTE, C.** It is Time to Individualize the Dialysate Sodium Prescription. *Seminars in Dialysis,* 2016, vol. 29, 24-27
- **MENDOZA, J. M ; SUN, S ; CHERTOW, G. M ; MORAN, J. ; DASS, S ; SCHILLER, B.** Dialysate Sodium and Sodium gradient in maintenance hemodialysis: a neglected sodium restriction approach?. *Nephrol Dial Transplant,* 2011, vol. 26, 1281-1287
- Zero Diffusive Sodium Balance in Hemodialysis Provided by an Algorithm-Based Electrolyte Balancing Controller: A Proof of Principle Clinical Study. **KUHLMANN, U ; MAIERHOFER, A ; CANAUD, B ; HOYER, J ; GROSS, M.** Artificial Organs. Wiley, 2018
- **PEIXOTO, A. J ; GOWDA, N ; PARIKH, C. R ; SANTOS, S. F. F.** Long-Term Stability of Serum Sodium in Hemodialysis Patients. *Blood Purif,* 2010, vol. 29, 264-267
- **KEEN, M. L ; GOTCH, F. A.** The association of the sodium setpoint to interdialytic weight gain and blood pressure in hemodialysis patients. *The International Journal of Artificial Organs,* 2007, vol. 30, 971-979
- **HECKING, M ; KAINZ, A ; HÖRL, W. H ; HERKNER, H ; SUNDER-PLASSMANN, G.** Sodium Setpoint and Sodium Gradient: Influence on Plasma Sodium Change and Weight Gain. *Am J Nephrol,* 2011, vol. 33, 39-48